# EUROPEAN PATENT APPLICATION

(11) **EP 2 340 724 A2**
(43) Date of publication of application: **06.07.2011**
(21) Application number: 11002915.4
(22) Date of filing: 04.01.2007
(51) Int. Cl.: A23L 1/29, A23L 1/305, A61K 9/00, A61P 29/00, A61K 31/198

(54) **Treatment of stressed patients**

(30) Priority: 09.01.2006 US 757835 P
(62) Divisional of application: 07703658.0
(71) Applicant: Nestec S.A., 1800 Vevey (CH)
(72) Inventor: Schiffrin, Eduardo, 1023 Crissier (CH); Grad, Teresa, St. Albert Alberta T8N 7C7 (CA); Murbach, François, 01220 Divonne (FR)
(74) Representative: Lock, Graham James

(57) **Abstract**

Methods and compositions for providing nutrition to critically ill patients are presented. In an embodiment, the method comprises administering to a patient undergoing a condition characterized by a systemic inflammatory response phase followed by a compensatory anti-inflammatory response phase a first nutritional composition while the patient is experiencing the first phase and administering to the patient a second different nutritional composition while the patient is experiencing the second phase.

## Description

### BACKGROUND

The present invention generally relates to health and nutrition. More specifically, the present invention relates to methods and compositions for providing nutrition to stressed or critically ill patients.

In a medical center's intensive care unit ("ICU"), stressed or critically ill patients are typically those with conditions such as sepsis, severe infection, acute pancreatitis, trauma and burns or patients having undergone large abdominal surgery. Initially these patients are in shock or stress and their metabolic response results in the production of free radicals and pro-inflammatory cytokines. In addition, blood flow to the gut region drops, metabolic rate drops and glutathione levels drop. The end results to the patient during this phase are increased oxidative stress, inflammation, hypercatabolism, hyperglycaemia and loss of gut integrity.

Once this phase is over, the patient enters a catabolic phase during which a compensatory anti-inflammatory response takes place. Typically energy expenditure increases and there is muscle protein loss. Further, the immune function is impaired and gut atrophy occurs. Generally, the patient leaves the ICU during this phase. Because these two phases affect the body differently, the patient can have very different nutritional needs during each phase.

### SUMMARY

The present invention generally relates to health and nutrition. More specifically, the present invention relates to methods and compositions for providing nutrition to critically ill patients such as, for example, patients in ICUs.

In an embodiment, the present invention provides a method for providing nutrition to a critically patient undergoing a condition characterized by a Systemic Inflammatory Response phase (hereinafter "SIRS") and a Compensatory Anti-Inflammatory Response phase (hereinafter "CARS"). For example, the method comprises administering to the patient a first nutritional composition while the patient is experiencing the SIRS phase and administering to the patient a second different nutritional composition while the patient is experiencing the CARS phase.

In an embodiment, the first nutritional composition comprises one or more components selected from the group consisting of a protein source, a carbohydrate source, a lipid source, vitamins, minerals and combinations thereof

In an embodiment, the protein source of the first nutritional composition comprises threonine. The protein source can also comprise a component selected from the group consisting of sweet whey protein, caseino-glyco-macropeptide, casein supplemented by free threonine, threonine-containing compound and combinations thereof. The threonine can comprise at least about 5.5% by weight of the amino acids of the protein source.

In an embodiment, the protein source of the first nutritional composition comprises cysteine. The protein source can also comprise a component selected from the group consisting of whey protein, free glutathione, cysteine precursors, prodrugs and combinations thereof.

In an embodiment, the protein source of the first nutritional composition comprises a hydrolyzed protein. The protein source can also comprise a hydrolyzed whey protein.

In an embodiment, the protein source provides about 10% to about 20% of the energy of the first nutritional composition.

In an embodiment, the carbohydrate source of the first nutritional composition comprises a component selected from the group consisting of maltodextrin, corn starch, modified starch, sucrose and combinations thereof. The carbohydrate source can provide about 35% to about 65% of the energy of the first nutritional composition.

In an embodiment, the first nutritional composition is free from lactose.

In an embodiment, the lipid source of the first nutritional composition comprises a mixture of medium chain triglycerides and long chain triglycerides. The lipid source can comprise at least about 30% to about 80% by weight of medium chain triglycerides. The lipid source can comprise a component selected from the group consisting of sunflower oil, rapeseed oil, soy oil, milk fat, corn oil, soy lecithin, fractionated coconut oil and combinations thereof.

In an embodiment, the first nutritional composition comprises a lipid profile designed to have a polyunsaturated fatty acid omega-6 (n-6) to omega-3 (n-3) ratio of about 1:1 to about 10:1.

In an embodiment, the lipid source provides about 20% to about 50% of the energy of the first nutritional composition.

In an embodiment, the first nutritional composition has an energy content of about 800 kcal/l to about 1200 kcal/l.

In an embodiment, the first nutritional composition is in the form of a soluble powder, a liquid concentrate, a ready-to-drink formulation and combinations thereof.

In an embodiment, the second nutritional composition comprises one or more components selected from the group consisting of a protein source, a lipid source, a carbohydrate source and combinations thereof. The protein source can comprise about 18% to about 28% of the energy of the second nutritional composition. The protein source can include a protein hydrolysate. The protein source can comprise a component selected from the group consisting of casein hydrolysate, whey hydrolysate and combinations thereof. The protein hydrolysate can comprise cysteine.

In a embodiment, the protein source of the second nutritional composition can comprise a portion as free amino acids. For example, the protein source can include approximately 80% to 85% of protein hydrolysate and approximately 15% to 20% of free amino acids.

In an embodiment, the lipid source comprises approximately 30% to about 45% of the energy of the second nutritional composition. The lipid source can comprise a mixture of medium chain triglycerides and long chain triglycerides. The lipid source can comprise about 30% to about 80% by weight of medium chain triglycerides.

In an embodiment, the lipid source of the second nutritional composition comprises a component selected from the group consisting of fractionated coconut oil, fish oil, sunflower oil, rapeseed oil, soy oil, milk fat, corn oil, soy lecithin and combinations thereof.

In an embodiment, the second nutritional composition contains omega-3 rich fatty acids.

In an embodiment, the second nutritional composition comprises a lipid profile designed to have a polyunsaturated fatty acid omega-6 (n-6) to omega-3 (n-3) ratio of about 1:1 to about 10:1.

In an embodiment, the carbohydrate source of the second nutritional composition provides about 30% to about 65% of the energy of the second nutritional composition. The carbohydrate source can comprise a component selected from the group consisting of maltodextrin, corn starch, modified starch, sucrose and combinations thereof.

In an embodiment, the second nutritional composition is free from lactose.

In an embodiment, the second nutritional composition is in the form of a soluble powder, a liquid concentrate, a ready-to-drink formulation and combinations thereof.

An advantage of the present invention is to provide an improved method for providing nutritional to critically ill patients such as, for example, patients in ICUs.

Another advantage of the present invention is to provide improved clinical nutrition products.

Additional features and advantages are described herein, and will be apparent from, the following Detailed Description.

### DETAILED DESCRIPTION

The present invention generally relates to health and nutrition. More specifically, the present invention relates to methods of and compositions for providing nutrition to critically ill patients.

In the present specification, the term "critically ill" should be understood to mean a patient undergoing a condition characterized by a SIRS phase and a CARS phase. Critically ill patients undergoing high stress or shock such as, for example, those in ICUs typically enter into a SIRS phase followed by a CARS phase. For instance, during the SIRS phase, the body can experience a systemic inflammatory response. This is characterised by increasing production of free radicals and pro-inflammatory cytokines coupled with decreasing glutathione levels, metabolic rate and blood flow to the gut which may result in oxidative stress, inflammation, hyperglycaemia and loss of gut integrity. The SIRS phase may be preceded by an initial ebb or shock phase characterised by low metabolic activity. This is followed by a flow phase which is a state characterised in terms of increasing metabolic activity accompanied initially by a peak in inflammatory activity and then declining inflammation as the SIRS phase gives way to the CARS phase. During the CARS phase, the body can experience a compensatory anti-inflammatory response characterised by hypercatabolism and hypermetabolism, impaired immune function hyperglycaemia and gut atrophy resulting in increasing energy expenditure, muscle protein loss and sepsis of gut origin.

In an embodiment, the present invention provides a method comprising providing one or more nutritional compositions to the patient that reduces the oxidative stress and promotes gut integrity followed by providing one or more different nutritional compositions to the patient that preserves lean body mass and reduces muscle breakdown. For example, prior to or during the SIRS phase, the patient can be fed a nutritional composition which reduces oxidative stress and promotes gut integrity. Prior to or during the following CARS phase, the patient can be fed a nutritional composition which preserves lean body mass and reduces muscle breakdown. Nutritionally managing the patients in this manner reduces the risk of infection and loss of body mass and increases the probability of the patients recovering, and recovering quicker.

In an embodiment, the nutritional compositions for the SIRS phase (hereinafter "NCSPs") ideally contain a protein source which contributes to reduction of oxidative stress and promotion of gut integrity. To promote gut integrity, a suitable protein source can be one that is naturally enriched in threonine such as, for example, sweet whey protein or caseino-glyco-macropeptide. Sweet whey protein has a threonine content of about 7.4% by weight of amino acids and caseino-glyco-macropeptide has a threonine content of about 14% by weight of amino acids.

It should be appreciated that the protein source may be any suitable protein such as, for example, casein supplemented by free threonine or any compound or source containing threonine. Preferably, threonine provides at least about 5.5% by weight of the amino acids of the protein source. More preferably, threonine provides at least about 6% by weight of the amino acids of the protein source. Threonine increases mucin synthesis in the gut and in consequence gut integrity.

To promote a reduction of oxidative stress, a suitable protein source can be one that is naturally enriched in cysteine such as, for example, whey protein. Administration of cysteine increases intracellular glutathione levels. Glutathione protects cells against free radicals, reactive oxygen intermediates and toxic compounds that are both of endogenous and of exogenous origin. See, Meister, "New Aspects of Glutathione Biochemistry and Transport-Selective Alteration of Glutathione Metabolism," Nutrition Review, 42:397-410. Alternatively, the protein source may include free glutathione or a cysteine precursor or prodrug such as, for example, that described in US Patent No. 5,863,906, which is hereby incorporated by reference.

The protein source is preferably based upon hydrolyzed protein. Especially preferred in this regard is hydrolyzed whey protein. Hydrolyzed protein supports gut integrity. The protein source may provide about 10% to about 20% of the energy of the nutritional composition. For example, the protein source may provide about 15% to about 18% of the energy of the nutritional composition.

In another embodiment, the NCSPs may also include a carbohydrate source. The carbohydrate source preferably provides about 35% to about 65% of the energy of the nutritional composition. More preferably, the carbohydrate source provides about 40% to 60% of the energy of the nutritional composition. For example, the carbohydrate source may provide about 51% of the energy of the composition. Several carbohydrates may be used including maltodextrin, corn starch, modified starch, sucrose or mixtures thereof Preferably the composition is free from lactose.

In an alternative embodiment, the NCSPs may further include a lipid source. Preferably, the lipid source provides about 20% to about 50% of the energy of the nutritional composition. More preferably, the lipid source provides about 25% to about 40% of the energy of the nutritional composition. For example, the lipid source may provide about 33% of the energy of the nutritional composition.

The lipid source may comprise a mixture of medium chain triglycerides (MCT) and long chain triglycerides (LCT). The lipid source preferably includes at least about 30% to about 80% by weight of medium chain triglycerides. For example, medium chain triglycerides may make up about 70% by weight of the lipid source. Suitable sources of long chain triglycerides are sunflower oil, rapeseed oil, soy oil, milk fat, corn oil and/or soy lecithin. Fractionated coconut oils are a suitable source of medium chain triglycerides.

The lipid profile of the enteral composition is preferably designed to have a polyunsaturated fatty acid omega-6 (n-6) to omega-3 (n-3) ratio of about 1:1 to about 10:1. For example, the n-6 to n-3 fatty acid ratio may be about 6:1 to about 9:1.

In another embodiment, the NCSPs preferably include a complete vitamin and mineral profile. For example, sufficient vitamins and minerals may be provided to supply about 75% to about 250% of a suitable recommended daily allowance of the vitamins and minerals per 1000 calories of the nutritional composition. Preferably, the NCSPs contains vitamins and minerals with antioxidant properties.

The NCSPs preferably have an energy content of about 800 kcal/l to about 1200 kcal/l. More preferably, the NCSPs have an energy content of about 1000 kcal/l.

It should be appreciated that the NCSPs may be in any suitable form. For example, the NCSPs may be in the form of a soluble powder, a liquid concentrate, and/or a ready-to-drink formulation.

By way of example and not limitation, the following examples are illustrative of various embodiments of the NCSPs. In an embodiment, a suitable NCSP is commercialized by Nestlé Nutrition under the brand name PEPTAMEN®. This composition includes, for example, the following characteristics:
- an energy density of 1.0 kcal/ml
- a protein source made up of hydrolyzed whey protein and which provides 16% of total energy
- a lipid source which contains 70% MCT's (fractionated coconut oil and palm kernel oil) and 30% LCT's
- a n6:n3 ratio of 7.4:1
- meets or exceeds 100% of the reference daily intake ("RDI") for 22 key vitamins and minerals in 1500 mL (1500 kcal)
- vitamins include A, B₁, B₂, B₆, B₁₂, niacin, folic acid, biotin, pantothenic acid, C, D, E, K, etc.; minerals include calcium, phosphorus, magnesium, zinc, iron, copper, manganese, iodine, sodium, potassium, chloride, chromium, molybdenum, selenium, etc.
- lactose-free and gluten-free
- maltodextrin, corn starch, soybean oil, soy lecithin

The nutritional compositions for the CARS phase (hereinafter "NCCPs") also comprises ingredients specifically tailored for treating the patient undergoing the catabolic phase. Preferably, the NCCPs have a high energy content. For example, the energy content can be between about 1.3 to about 1.5 kcal/ml or higher. Providing a moderate-to-high energy intake is important to spare muscle protein.

Additionally, due to increased metabolic activity, the protein content of the NCCPs is raised. For example, the protein content may be to provide about 18% to about 28% of the energy of the product. The protein source preferably includes a protein hydrolysate. The use of protein hydrolysate reduces the potential for nutrient malabsorption. A variety of hydrolyzed proteins may be utilized. Suitable examples include casein hydrolysate and whey hydrolysate. The hydrolysate source also preferably includes a sufficient amount of cysteine to replenish intracellular glutathione in the patient.

The protein source of the NCCPs may further include a portion as free amino acids. For example, the protein source may be enriched with arginine. Preferably, the protein source includes approximately 80% to 85% of protein hydrolysate and approximately 15% to 20% of free amino acids.

The NCCPs may further include a lipid source. The formulation of the can also include a lipid fraction. Preferably, approximately 30% to about 45% of the formulation, by energy, is provided as a lipid.

The lipid source of the NCCPs may comprise a mixture of medium chain triglycerides ("MCT") and long chain triglycerides ("LCT"). The lipid source preferably includes at least about 30% to about 80% by weight of medium chain triglycerides. For example, medium chain triglycerides may make up about 50% to 70% by weight of the lipid source. MCTs are more easily absorbed and metabolized as compared to LCTs. Therefore, the use of MCTs will increase energy uptake and hence may spare nitrogen in the body for anabolism. Fractionated coconut oils are a suitable source of medium chain triglycerides. Suitable sources of long chain triglycerides are fish oil, sunflower oil, rapeseed oil, soy oil, milk fat, corn oil and/or soy lecithin.

The LCT portion of the lipid source contains omega-3 rich fatty acids to reduces the incidence and severity of inflammatory reactions. Omega-3 fatty acids may modulate the negative, immune-mediated reactions brought about by high omega-6 intake. Therefore, oil blends that contain omega-3 (or are, at a minimum, low in omega-6) are preferred. The lipid profile of the enteral composition is preferably designed to have a polyunsaturated fatty acid omega-6 (n-6) to omega-3 (n-3) ratio of about 1:1 to about 10:1. For example, the n-6 to n-3 fatty acid ratio may be about 1:1 to about 3:1.

Fish oil rich in omega-3 fatty acids is preferred, as fish oils contain two longer chain length omega-3 fatty acids: eicosapentaenoic acid (EPA, C22:5, n3) and docosahexaenoic acid (DHA, C22:6, n3).

The NCCPs may also include a carbohydrate source. The carbohydrate source preferably provides about 30% to about 65% of the energy of the nutritional composition. More preferably, the carbohydrate source provides about 35% to 60% of the energy of the nutritional composition. Several carbohydrates may be used including maltodextrin, corn starch, modified starch, sucrose or mixtures thereof. Preferably, the composition is free from lactose.

It should be appreciated that the NCCPs may be in any suitable form. For example, the NCCPs may be in the form of a soluble powder, a liquid concentrate, and/or a ready-to-drink formulation.

By way of example and not limitation, the following examples are illustrative of various embodiments of the NCCPs. In an embodiment, a suitable NCCP is commercialized by Nestlé Nutrition under the brand name CRUCIAL®. This composition includes, for example, the following characteristics:
- a high energy density of 1.5 kcal/ml,
- hydrolyzed protein (hydrolyzed casein) as protein source which contributes 25% of the energy of the composition (the protein source also contains arginine)
- a lipid blend of marine oil, MCT (fractionated coconut oil and palm kernel oil) and soy oil with a n6:n3 ratio of 1.5:1
- elevated levels of Beta-Carotene, Vitamin A, Vitamin C and Zinc
- meets or exceeds 100% of the RDI for 23 key vitamins and minerals in 1000 mL (1500 kcal)
- vitamins include A, B₁, B₂, B₆, B₁₂, niacin, folic acid, biotin, pantothenic acid, C, D, E, K, etc.; minerals include calcium, phosphorus, magnesium, zinc, iron, copper, manganese, iodine, sodium, potassium, chloride, chromium, molybdenum, selenium, etc.
- a moderate osmolality (490 mOsm/kg H₂O)
- lactose-free, gluten-free and low-cholesterol
- maltodextrin, fish oil, corn starch, soy lecithin

Another suitable NCCP is commercialized by Nestlé Nutrition under the brand name PEPTAMEN® 1.5. This composition includes, for example, the following characteristics:
- energy density of 1.5 kcal/ml
- a protein source made up of hydrolyzed whey protein and which provides 18% of total energy
- a lipid source which contains 70% MCT's (fractionated coconut oil and palm kernel oil) and 30% LCT's, has a n6:n3 ratio of 7.4:1
- meets or exceeds 100% of the RDI for 22 key and minerals in 1500 mL (1500 kcal)
- vitamins include A, B₁, B₂, B₆, B₁₂, niacin, folic acid, biotin, pantothenic acid, C, D, E, K, etc.; minerals include calcium, phosphorus, magnesium, zinc, iron, copper, manganese, iodine, sodium, potassium, chloride, chromium, molybdenum, selenium, etc.
- lactose-free and gluten-free
- maltodextrin, corn starch, soybean oil, soy lecithin

It should be understood that various changes and modifications to the presently preferred embodiments described herein will be apparent to those skilled in the art. Such changes and modifications can be made without departing from the spirit and scope of the present subject matter and without diminishing its intended advantages. It is therefore intended that such changes and modifications be covered by the appended claims.

## Claims

1. A method of providing nutrition to a critically ill patient undergoing a condition **characterized by** a SIRS phase and a CARS phase, the method comprising:
administering to the patient a first nutritional composition while the patient is experiencing the SIRS phase; and
administering to the patient a second different nutritional composition while the patient is experiencing the CARS phase.

2. The method of Claim 1, wherein the first nutritional composition comprises at least one component selected from the group consisting of a protein source, a carbohydrate source, a lipid source, vitamins, minerals and combinations thereof.

3. The method of Claim 2, wherein the protein source comprises (a) threonine; (b) a component selected from the group consisting of sweet whey protein, caseino-glyco-macropeptide, casein supplemented by free threonine, threonine-containing compound and combinations thereof; (c) cysteine; (d) a component selected from the group consisting of whey protein, free glutathione, cysteine precursors, prodrugs and combinations thereof; (e) a hydrolyzed protein; (f) a hydrolyzed whey protein.

4. The method of Claim 2, wherein the carbohydrate source comprises a component selected from the group consisting of maltodextrin, corn starch, modified starch, sucrose and combinations thereof.

5. The method of Claim 2, wherein the first nutritional composition is free from lactose.

6. The method of Claim 2, wherein the lipid source comprises (a) a mixture of medium chain triglycerides and long chain triglycerides; optionally wherein the lipid source comprises at least about 30% to about 80% by weight of the medium chain triglycerides; or (b) a component selected from the group consisting of sunflower oil, rapeseed oil, soy oil, milk fat, corn oil, soy lecithin, fractionated coconut oil and combinations thereof.

7. The method of Claim 2, wherein the first nutritional composition comprises a lipid profile designed to have a polyunsaturated fatty acid omega-6 (n-6) to omega-3 (n-3) ratio of about 1:1 to about 10:1.

8. The method of Claim 2, wherein the first nutritional composition is in the form of a soluble powder, a liquid concentrate, a ready-to-drink formulation and combinations thereof.

9. The method of Claim 1, wherein the second different nutritional composition comprises at least one component selected from the group consisting of a protein source, a lipid source, a carbohydrate source and combinations thereof; optionally wherein (a) the protein source includes (i) a protein hydrolysate; optionally wherein the protein hydrolysate comprises cysteine; or (ii) a component selected from the group consisting of casein hydrolysate, whey hydrolysate and combinations thereof; or (iii) a portion as free amino acids; or (b) the lipid source comprises (i) a mixture of medium chain triglycerides and long chain triglycerides; or (ii) a component selected from the group consisting of fractionated coconut oil, fish oil, sunflower oil, rapeseed oil, soy oil, milk fat, corn oil, soy lecithin and combinations thereof; or (iii) omega-3 rich fatty acids; or (c) the second nutritional composition comprises a lipid profile designed to have a polyunsaturated fatty acid omega-6 (n-6) to omega-3 (n-3) ratio of about 1:1 to about 10:1; or (d) the carbohydrate source comprises a component selected from the group consisting of maltodextrin, corn starch, modified starch, sucrose and combinations thereof; or (e) the second nutritional composition is free from lactose; or (f) the second nutritional composition is in the form of a soluble powder, a liquid concentrate, a ready-to-drink formulation and combinations thereof.

10. A method for providing nutrition to a critically ill patient, the method comprising:
administering to the patient a first nutritional composition during a SIRS phase of the patient, wherein the first nutritional composition comprises a protein source including threonine, a carbohydrate source, a lipid source, vitamins and minerals; and
administering to the patient a second nutritional composition during a CARS phase of the patient, wherein the second different nutritional composition comprises a protein source having protein hydrolysates and free amino acids, a lipid source and a carbohydrate source.

11. A method for providing nutrition to a critically ill patient, the method comprising:
administering to the patient a first nutritional composition while the patient is experiencing a SIRS phase resulting from the patient's systemic inflammatory response to the stress, wherein the first nutritional composition comprises a protein source having threonine, a protein source having cysteine and a lipid source having a mixture of medium chain triglycerides and long chain triglycerides; and
administering to the patient a second nutritional composition while the patient is experiencing a CARS phase resulting from the patient's compensatory anti-inflammatory response to the stress, wherein the second different nutritional composition comprises a protein source having a protein hydrolysate and a protein source having a portion as free amino acids.
